# EUROPEAN PATENT APPLICATION

(11) **EP 4 417 153 A1**
(43) Date of publication of application: **21.08.2024**
(21) Application number: 23157790.9
(22) Date of filing: 21.02.2023
(51) Int. Cl.: A61B 18/14, A61B 17/29

(54) **HEMOSTATIC FORCEPS FOR ENDOSCOPE**

(30) Priority: 15.02.2023 US 202318169644
(71) Applicant: Finemedix Co., Ltd., Daegu 41059 (KR)
(72) Inventor: JUNG, Young Geun, 41075 Dong-gu Daegu (KR)
(74) Representative: Stöckeler, Ferdinand

(57) **Abstract**

A hemostatic forceps includes a handle part, a slider (200) slidably installed on the handle part, a fluid injection part (300) connected to the handle part and having a fluid injection port, an outer tube extending from the fluid injection part, a housing coupled to an end of the outer tube, a pair of pincer pieces (600) installed on the housing so as to be openable and closable relative to each other, a pair of wires (700) each having two opposite ends respectively connected to the pair of pincer pieces and the slider, and an inner tube (800) disposed in the outer tube and configured to surround the pair of wires.

## Description

### BACKGROUND OF THE DISCLOSURE

### Field of the Disclosure

The present disclosure relates to hemostatic forceps for an endoscope, and more particu larly, to hemostatic forceps for an endoscope that may facilitate a rotation and swing of a pair of pincer pieces and inject a fluid.

### Description of the Related Art

A therapeutic endoscopy field using an endoscope has been considered an important field in a treatment area of gastroenterology. The therapeutic endoscopy using an electron microscope, i.e., an endoscopic treatment has been developed by virtue of the appearance of charge coupled devices (CCD), the miniaturization of electronic elements, the development of image technologies, various diagnoses through working channels, the development of treatment technologies, and the deliberative skill of treatment practitioners.

For example, in the past, the treatment of cancer such as gastric cancer or colorectal cancer depended on a surgical method. However, recently, by virtue of the appearance of the endoscope, endoscopic submucosal dissection (ESD) has been performed by using a treatment device inserted into a working channel provided in the endoscope, and thus clinical diagnosis and treatment have been performed at the same time, such that a rate of an endoscopic treatment of early cancer such as early gastric cancer or early colorectal cancer has increased.

During the procedure of the endoscopic submucosal dissection or endoscopic mucosal resection (EMR), various types of treatment tools are used for the treatment device inserted through the working channel of the endoscope.

Typically, there are used various types of treatment devices such as a knife used to incise a mucous membrane of a tissue, a coagulator used to arrest bleeding in the event of hemorrhage during a process such as the incision of the mucous membrane, a needle used for injecting a medicine that is required to inject a saline solution or the like as a means for expanding tissue to facilitate the incision, and a treatment device used to inject a washer fluid for easily ensuring a visual field blocked by hemorrhage or the like.

Korean Patent Application Laid-Open No. 2012-0026537 discloses high-frequency hemostatic forceps 20 for an endoscope in the related art.

An overall configuration of the hemostatic forceps 20 will be described. A pair of forcep cups 1, which serves as a high-frequency electrode, is installed at a tip of a flexible sheath 2. In this case, the flexible sheath 2 is formed by covering a tightly wound coil 7 with an electrically insulating flexible tube 8. A slider 5 is disposed on an operation part 3 connected to an end (base portion) at a side of a handle of the flexible sheath 2. The slider 5 is an operation member that slides a conductive operation wire 4, which passes through the flexible sheath 2, in a longitudinal direction. Therefore, when the slider 5 operates to slide the operation wire 4 in the longitudinal direction, the pair of forcep cups 1 may be opened forward or closed with respect to a center of a support shaft 12.

However, in the related art, because the operation wire 4 is bent when the slider 5 moves forward, the pair of forcep cups 1 is not immediately opened, and the pair of forcep cups 1 does not rotate and swing. Even though the pair of forcep cups 1 may rotate, the operation wire 4 needs to be twisted to some extent in order to transmit a rotation of the operation part 3 through the operation wire 4 to the pair of forcep cups 1 positioned at a long distance and provide a rotational force to the pair of forcep cups 1, which causes a problem in that a rotation reaction is slow and difficult to precisely adjust.

In addition, there is a problem in that a separate device for injecting a fluid is required to ensure a visual field when it is difficult to accurately identify a hemorrhage site.

### SUMMARY OF THE DISCLOSURE

An object of the present disclosure is to provide hemostatic forceps for an endoscope that may facilitate a rotation and swing of a pair of pincer pieces and inject a fluid.

Technical problems to be solved by the present disclosure are not limited to the above-mentioned technical problems, and other technical problems, which are not mentioned above, may be clearly understood from the following descriptions by those skilled in the art to which the present disclosure pertains.

An embodiment of the present disclosure provides hemostatic forceps for an endoscope, the hemostatic forceps including: a handle part; a slider slidably installed on the handle part; a fluid injection part connected to the handle part and having a fluid injection port; an outer tube extending from the fluid injection part; a housing coupled to an end of the outer tube; a pair of pincer pieces installed on the housing so as to be openable and closable relative to each other; a pair of wires each having two opposite ends respectively connected to the pair of pincer pieces and the slider; and an inner tube disposed in the outer tube and configured to surround the pair of wires.

According to the embodiment, the housing may include: a first member coupled to an end of the outer tube; and a second member rotatably coupled to the first member, and the pair of pincer pieces may be installed on the second member so as to be openable and closable relative to each other.

According to the embodiment, the second member may be rotatably coupled to the first member through a connection member, one end of the connection member may be fixedly coupled to an inner side of the second member, and the other end of the connection member may be coupled to an inner stepped portion of the first member.

According to the embodiment, the second member may include a plurality of hook parts protruding toward the first member, and the plurality of hook parts may be coupled to an inner stepped portion of the first member.

According to the embodiment, the inner stepped portion of the first member may be formed on an inner surface of the first member so that a height of the inner stepped portion increases in a direction toward the second member.

According to the embodiment, a surface of the first member and a surface of the second member, which face each other in an axial direction, may be spaced apart from each other.

According to the embodiment, the inner tube may be disposed to be spaced apart from an end of the housing at a predetermined distance.

According to the embodiment, a fluid injected through the fluid injection port flows through a space between the outer tube and the inner tube and is sprayed through a hollow portion of the housing.

According to the embodiment, a coil may be wound around an inner surface of the outer tube.

According to the embodiment, a protection tube may be coupled to an end of the fluid injection part and configured to surround the outer tube.

Another embodiment of the present disclosure provides hemostatic forceps for an endoscope, the hemostatic forceps including: a handle part; a slider slidably installed on the handle part; a rotary dial part connected to the handle part; an outer tube extending from the rotary dial part; a housing coupled to an end of the outer tube; a pair of pincer pieces installed on the housing so as to be openable and closable relative to each other; a pair of wires each having two opposite ends respectively connected to the pair of pincer pieces and the slider; and an inner tube disposed in the outer tube and configured to surround the pair of wires.

According to the embodiment, the rotary dial part includes: a holder part fixedly coupled to the handle part and having a pair of holder arms spaced apart from each other; a pipe connected to the pair of holder arms of the holder part while penetrating the pair of holder arms; and a rotary dial rotatably disposed between the pair of holder arms of the holder, and in which the pipe is disposed in the rotary dial.

According to the embodiment, the rotary dial may have a through-hole in a longitudinal direction, and the pipe may be disposed in the through-hole and rotates together with the rotary dial when the rotary dial rotates.

According to the embodiment, the through-hole of the rotary dial may have a polygonal shape, and a cross-section of the pipe may have a polygonal shape substantially identical to the polygonal shape of the through-hole.

According to the embodiment, the pair of wires may be connected to the pipe and rotated together with the pipe by a rotation of the pipe and be configured to rotate the pair of pincer pieces coupled to distal ends of the pair of wires.

According to the present disclosure, the forceps may include the inner tube that moves forward or rearward while surrounding the pair of wires, thereby preventing the situation in which the pair of wires is bent and interferes with the inner surface of the outer tube when the slider moves the pair of wires forward or rearward. Therefore, the pair of wires may easily move forward or rearward.

In addition, the rotational force of the handle part may be immediately transmitted to the pair of pincer pieces even though the pair of wires is not twisted by the inner tube when the handle part is rotated and the rotational force is transmitted to the pair of pincer pieces through the pair of wires.

Further, because the inner tube is disposed to be spaced apart from the end of the housing at a predetermined distance, i.e., because the inner tube does not surround a part of the front portion of each of the pair of wires, the wires may smoothly swing while being freely bent in the space in which the wires are spaced apart from the outer tube.

In addition, the fluid injected through the fluid injection port may flow through the space between the outer tube and the inner tube and then be sprayed through the hollow portion of the housing, and the sprayed fluid may flow along the outer surfaces of the pair of pincer pieces and then be easily sprayed to the hemorrhage site.

The effects of the present disclosure are not limited to the above-mentioned effects, and it should be understood that the effects of the present disclosure include all effects that may be derived from the configuration of the present disclosure disclosed in the detailed description of the present disclosure or the appended claims.

### BRIEF DESCRIPTION OF THE DRAWINGS

FIG. 1 is a side view illustrating hemostatic forceps for an endoscope according to an embodiment of the present disclosure.
FIG. 2 is a partially cross-sectional view of the hemostatic forceps for an endoscope in FIG. 1.
FIG. 3 is an enlarged side view of a front portion in FIG. 1.
FIG. 4 is a side view illustrating a state in which a pair of pincer pieces in FIG. 3 is closed.
FIG. 5 is an exploded perspective view illustrating that an outer tube in FIG. 3 is removed and a housing is separated.
FIG. 6 is a cross-sectional view illustrating a state in which the housing in FIG. 5 is coupled.
FIG. 7 is a perspective view illustrating that the outer tube is removed from the front portion in FIG. 1.
FIG. 8 is a cross-sectional view of the front portion in FIG. 1.
FIG. 9 is an exploded perspective view illustrating hemostatic forceps for an endoscope according to another embodiment of the present disclosure from which a housing is separated.
FIG. 10 is a cross-sectional view illustrating a state in which the housing in FIG. 9 is coupled.
FIG. 11 is a side view illustrating hemostatic forceps for an endoscope according to still another embodiment of the present disclosure.
FIG. 12 is a cross-sectional view taken along line A-A' in FIG. 11 and illustrating a rotary dial part of the hemostatic forceps for an endoscope.

### DETAILED DESCRIPTION OF THE DISCLOSURE

Hereinafter, exemplary embodiments of hemostatic forceps for an endoscope of the present disclosure will be described with reference to the accompanying drawings.

In addition, the terms used below are defined considering the functions in the present disclosure and may vary depending on the intention of a user or an operator or a usual practice. The following embodiments are not intended to limit the protection scope of the present disclosure but just exemplary constituent elements disclosed claims in the present disclosure.

A part irrelevant to the description will be omitted to clearly describe the present disclosure, and the same or similar constituent elements will be designated by the same reference numerals throughout the specification. Throughout the specification, unless explicitly described to the contrary, the word "comprise/include" and variations such as "comprises/includes" or "comprising/including" will be understood to imply the inclusion of stated elements, not the exclusion of any other elements.

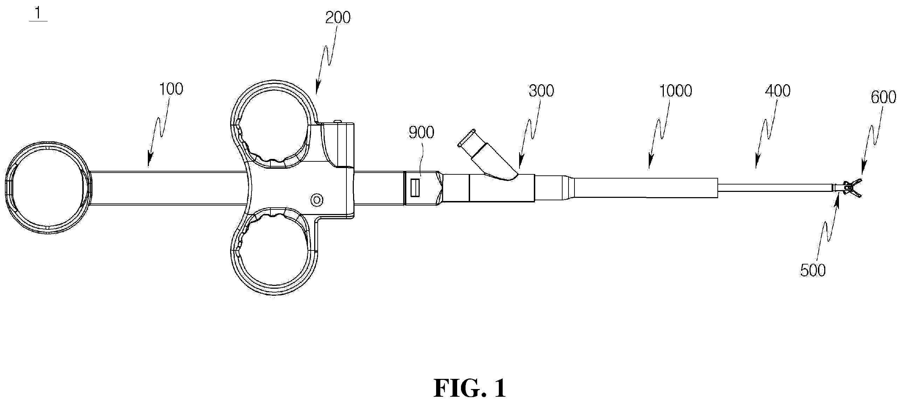

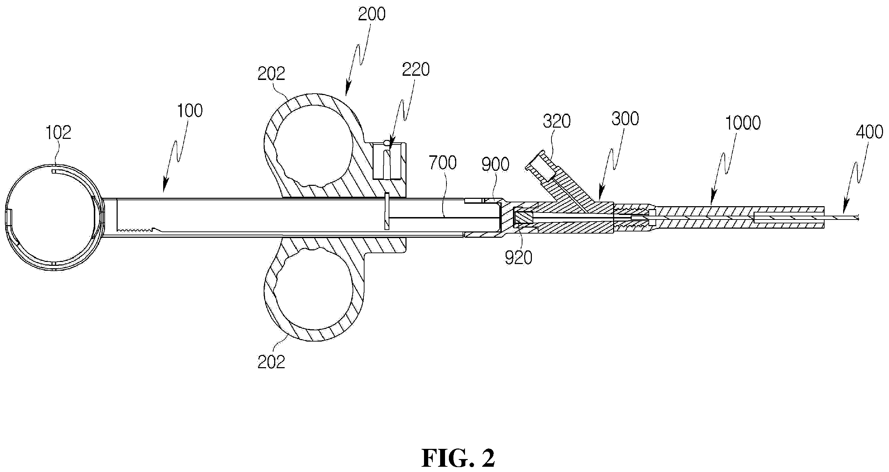

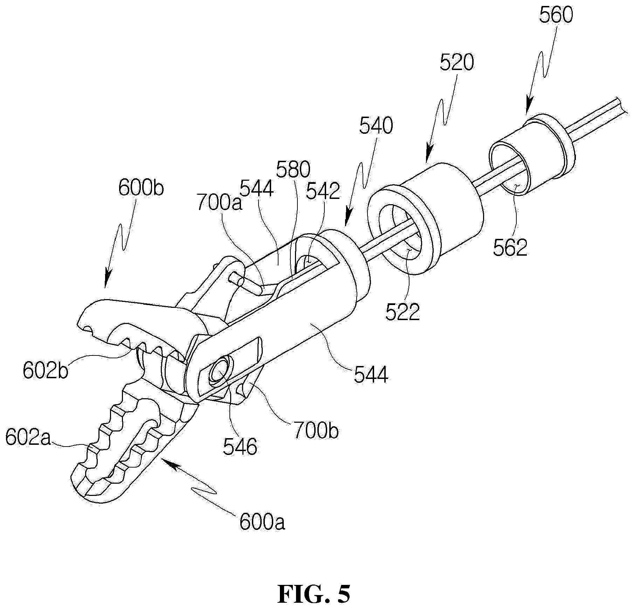

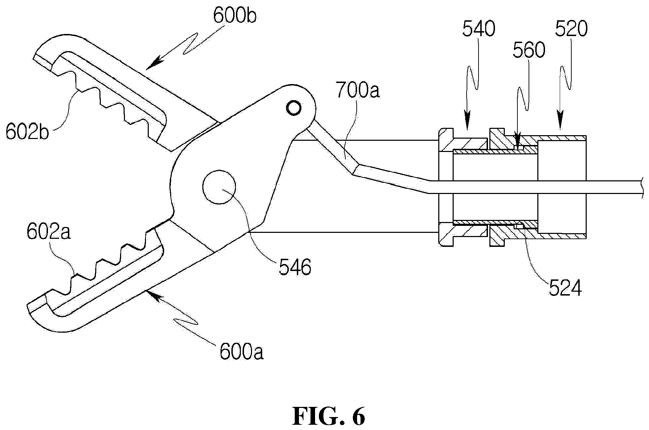

First, hemostatic forceps 1 for an endoscope according to an embodiment of the present disclosure will be described with reference to FIGS. 1 to 8.

The hemostatic forceps 1 for an endoscope according to the embodiment of the present disclosure may broadly include a handle part 100, a slider 200, a fluid injection part 300, an outer tube 400, a housing 500, a pair of pincer pieces 600, a pair of wires 700, and an inner tube 800.

The handle part 100 defines a body of the hemostatic forceps for an endoscope and has an elongated rod shape having a hollow portion. A cross-section of the handle part 100 may have any shape such as a circular shape, a quadrangular shape, or a triangular shape.

The slider 200 is slidably installed on the handle part 100. For example, a movement hole may be formed in a part of the handle part 100. The movement hole extends in a longitudinal direction and is formed through the handle part 100 in a radial direction. The slider 200 may be installed to penetrate the movement hole of the handle part 100, such that the slider 200 may slide in the longitudinal direction in the movement hole. A friction groove or the like may be formed in an outer surface of a part of the handle part 100 to prevent the slider 200 from being moved relative to the handle part 100 by a low force.

A handle ring 102 is provided at one end of the handle part 100, and slider rings 202 may be provided at two opposite sides of the slider 200. Therefore, a user may easily operate the slider 200 while holding the hemostatic forceps for an endoscope by inserting the fingers into the handle ring 102 and the slider rings 202. That is, the user may fix the thumb by inserting the thumb into the handle ring 102 and inserting the index finger and the middle finger into the slider rings 202, and the user may move the slider 200 forward or rearward relative to the handle part 100 depending on the motions of the index finger and the middle finger.

Hereinafter, a left side based on FIG. 1, i.e., a side at which the handle part 100 is provided will be defined and described as a rear side, and a right side, i.e., a side at which the pair of pincer pieces 600 is provided will be defined and described as a front side. Therefore, a situation in which the slider 200 moves toward the pair of pincer pieces 600 is a forward movement, and a situation in which the slider 200 moves away from the pair of pincer pieces 600 is a rearward movement.

The pair of wires 700 is coupled to the slider 200 and moves forward or rearward together with the slider 200, which will be described in more detail below. The slider 200 has a plug 220, and a high-frequency connection line or the like may be connected to the plug 220 so that the slider 200 receives high-frequency waves from a high-frequency electric generator that is separately used for medical purposes. The plug 220 may extend from one side from the slider 200 to the hollow portion of the handle part 100 and be connected to the pair of wires 700 disposed in the hollow portion of the handle part 100, thereby supplying the high-frequency waves. Therefore, when high-frequency current is applied, the high-frequency current may be transmitted to the pair of pincer pieces 600 connected to the plug 220 through the pair of wires 700.

The fluid injection part 300 includes a fluid injection port 320 coupled to a front end of the handle part 100 and having a tubular shape protruding to receive a fluid such as water, a saline solution, or the like from outside to inside. The fluid injection part 300 has a hollow portion, and the fluid injected through the fluid injection port 320 flows in the hollow portion in the fluid injection part 300. The pair of wires 700 extends from the hollow portion of the handle part 100 to the hollow portion of the fluid injection part 300 while penetrating the hollow portion of the handle part 100 and the hollow portion of the fluid injection part 300.

In the present embodiment, the fluid injection part 300 is connected to the handle part 100 through a separate connector 900. One end of the hollow portion in the fluid injection part 300 may be blocked by the connector 900. To prevent a leak of the fluid, a sealing part 920 may be disposed at a portion where the fluid injection part 300 and the connector 900 are connected to each other. In addition, the handle part 100 may be rotatably coupled to the connector 900. Therefore, the entire handle part 100 may rotate in a state in which the connector 900 is fixed.

The outer tube 400 extends forward from the fluid injection part 300. Therefore, the fluid injected into the fluid injection part 300 may flow into the outer tube 400. As described below, the fluid may flow forward through a space between the outer tube 400 and the inner tube 800.

In the present embodiment, a protection tube 1000 is coupled to a front end of the fluid injection part 300 and configured to surround the outer tube 400. The protection tube 1000 may be made of a material having higher rigidity than a material of the outer tube 400. Therefore, the protection tube 1000 may protect the outer tube 400 and prevent buckling. For example, the protection tube 1000 may be configured as a Teflon tube, and the outer tube 400 may be configured as a tube made of resin or the like and having insulation and flexibility.

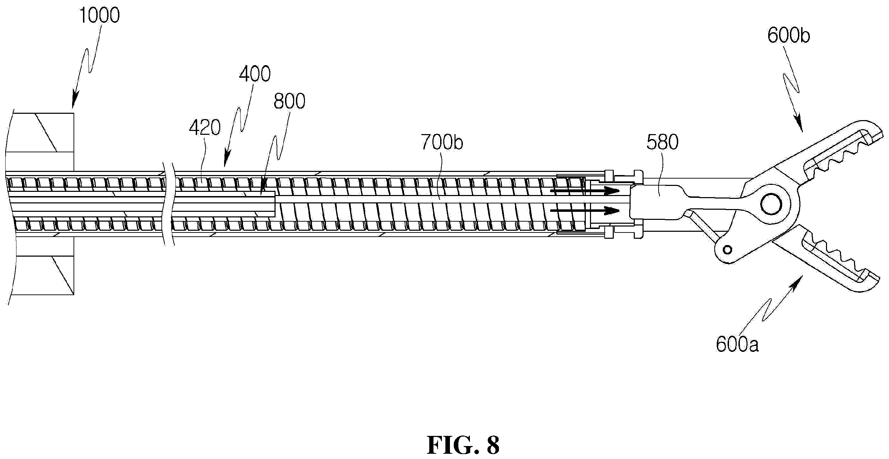

The outer tube 400 may be coupled to an interior of the fluid injection part 300 and extend forward. Alternatively, the outer tube 400 may be coupled to an interior of the protection tube 1000 and extend forward. In addition, as illustrated in FIG. 8, a coil 420 may be wound around an inner surface of the outer tube 400.

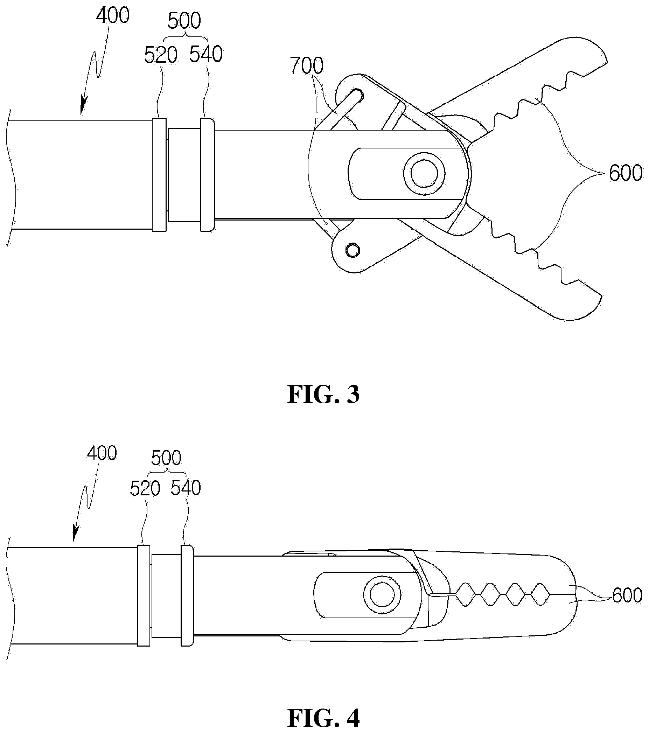

As illustrated in FIGS. 3 to 6, the housing 500 is coupled to a front end of the outer tube 400, and the pair of pincer pieces 600 is openably and closably installed on the housing 500. The housing 500 serves to support the pair of pincer pieces 600 so that the pair of pincer pieces 600 is rotatable and openable or closable relative to each other.

Specifically, the housing 500 includes: a first member 520 coupled to the front end of the outer tube 400; and a second member 540 coupled to be rotatable relative to the first member 520, and the pair of pincer pieces 600 is installed on the second member 540 so as to be openable and closable relative to each other. In the present embodiment, the second member 540 is rotatably coupled to the first member 520 through a connection member 560.

The first member 520 has a cylindrical shape having a first hollow portion 522 and is fixed to the front end of the outer tube 400. The second member 540 also has a cylindrical shape having a second hollow portion 542 and includes a pair of arms 544 extending outward from a cylindrical body while facing each other. The pair of pincer pieces 600 is installed on the pair of arms 544 so as to be openable and closable relative to each other. Specifically, the pair of pincer pieces 600 is installed to be rotatable side by side by a pin 546 installed to simultaneously penetrate the pair of arms 544.

The connection member 560 also has a cylindrical shape having a third hollow portion 562. One end of the connection member 560 is fixedly coupled to an inner side of the cylindrical body of the second member 540 by welding or the like, and the other end of the connection member 560 is coupled to a stepped portion 524 formed on an inner surface of the first member 520. The stepped portion 524 may be formed on the inner surface of the first member 520 so that a height of the stepped portion 524 increases forward. Because the other end of the connection member 560 is coupled to the stepped portion 524, the second member 540 may be rotatably supported without separating forward from the first member 520.

In this case, a surface of the first member 520 and a surface of the second member 540, which face each other in an axial direction, may be spaced apart from each other. Therefore, friction may be minimized when the second member 540 rotates relative to the first member 520.

The pair of pincer pieces 600 includes a first pincer piece 600a and a second pincer piece 600b respectively having closing surfaces that are configured to engage with each other. Specifically, the first pincer piece 600a has a first closing surface 602a disposed at a lower side, and the second pincer piece 600b has a second closing surface 602b disposed at an upper side and configured to engage with the first closing surface 602a. The first closing surface 602a and the second closing surface 602b may each have a serrated shape. As illustrated in FIG. 4, when the pair of pincer pieces 600 is closed, crest portions of the serrated shapes may engage with one another at the same position. However, the present disclosure is not limited thereto. When the pair of pincer pieces 600 is closed, the crest portions of the serrated shapes of the first and second closing surfaces 602a and 602b may, of course, alternately engage with one another. Therefore, when a hemostasis site is positioned between the pair of pincer pieces 600 and then the pair of pincer pieces 600 is closed by pulling the slider 200, the hemostasis site may be cauterized and coagulated by applied high-frequency current, such that the hemostasis may be quickly performed.

In this case, the pair of wires 700 is connected to the pair of pincer pieces 600 and configured to open or close the pair of pincer pieces 600. Specifically, the pair of wires 700 includes: a first wire 700a having two opposite ends respectively connected to the first pincer piece 600a and the slider 200; and a second wire 700b having two opposite ends respectively connected to the second pincer piece 600b and the slider 200. A separating plate 580 may extend from the interior of the housing 500 toward the pair of pincer pieces 600 and configured to allow the first wire 700a and the second wire 700b to be spaced apart from each other without interfering with each other. The first wire 700a and the second wire 700b may be disposed at different sides based on the separating plate 580, thereby making it possible to prevent interference between the first wire 700a and the second wire 700b.

Therefore, when the slider 200 moves rearward, the pair of wires 700 connected to the slider 200 moves rearward together with the slider 200, such that the pair of pincer pieces 600 is closed. When the slider 200 moves forward, the pair of wires 700 connected to the slider 200 moves forward together with the slider 200, such that the pair of pincer pieces 600 is opened.

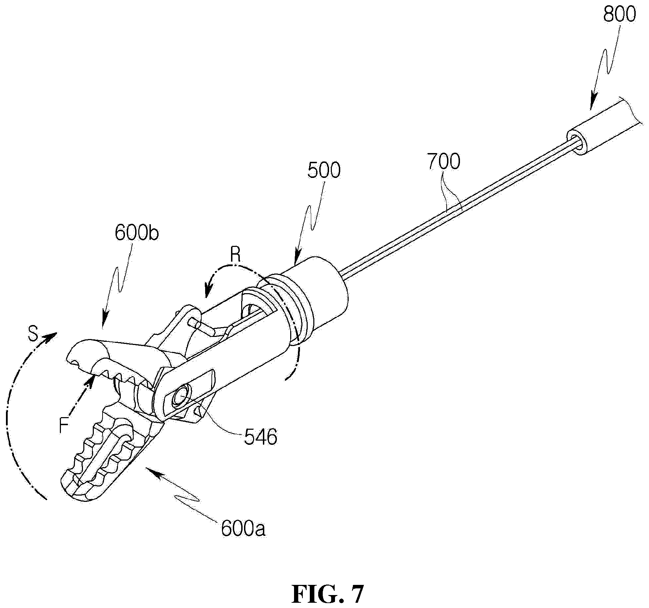

In the present disclosure, as illustrated in FIGS. 7 and 8, the hemostatic forceps include the inner tube 800 disposed in the outer tube 400 and configured to surround the pair of wires 700 so that the slider 200 may easily move the pair of wires 700 forward or rearward.

Because the inner tube 800 moves forward or rearward together with the pair of wires 700 while surrounding the pair of wires 700, the straightness of the pair of wires 700 may be maintained, such that not only the rearward movement but also the forward movement may be easily performed. That is, it is possible to prevent a situation in which the pair of wires 700 is bent and interferes with the inner surface of the outer tube 400 during the forward movement.

Moreover, a rotational force of the handle part 100 may be quickly transmitted to the pair of pincer pieces 600 even though the pair of wires 700 is not twisted by the inner tube 800 when the handle part 100 is rotated relative to the fluid injection part 300 and the rotational force is transmitted to the pair of pincer pieces 600 through the pair of wires 700 connected to the handle part 100.

Specifically, when the user holds the fluid injection part 300 and rotates the handle part 100, the handle part 100, the slider 200, the pair of wires 700 connected to the slider 200, the inner tube 800, and the pair of pincer pieces 600 are rotated in a state in which the fluid injection part 300 and the outer tube 400 connected to the fluid injection part 300 are fixed. In this case, because the second member 540 of the housing 500 is coupled to be rotatable relative to the first member 520 (direction R in FIG. 7), the pair of pincer pieces 600 may immediately rotate while corresponding to the rotation of the handle part 100.

The inner tube 800 may be disposed to be spaced apart from an end of the housing 500, particularly, an end of the first member 520 at a predetermined distance. That is, the inner tube 800 does not surround a part of a front portion of each of the pair of wires 700.

Therefore, in a section in which the inner tube 800 is not disposed, the pair of wires 700 may be freely unbent or bent in a space in which the pair of wires 700 is spaced apart from the outer tube 400. Therefore, a swing operation of rotating all the pair of pincer pieces 600 about the pin 546 may be smoothly performed (direction S in FIG. 7). For example, when a force F is generated and applied to the second pincer piece 600b from inside to outside depending on a hemorrhage position, the pair of pincer pieces 600 rotates in a direction of the second pincer piece 600b as a whole in a state in which the pair of pincer pieces 600 is maximally opened.

A route through which the fluid injected through the fluid injection port 320 is sprayed will be described. The fluid flows forward from the hollow portion of the fluid injection part 300 through the space between the outer tube 400 and the inner tube 800 and then is sprayed through the hollow portion of the housing 500 (see the arrows in FIG. 8. The fluid sprayed through the hollow portion of the housing 500 may meet the pair of pincer pieces 600 and flow along the outer surfaces of the pincer pieces, and the fluid may be accurately sprayed to the hemorrhage site, thereby making it possible to ensure a visual field. In particular, when the pair of pincer pieces 600 is closed, the fluid may be collected at the center of the pair of pincer pieces 600, such that the fluid may be more accurately sprayed to the hemorrhage site.

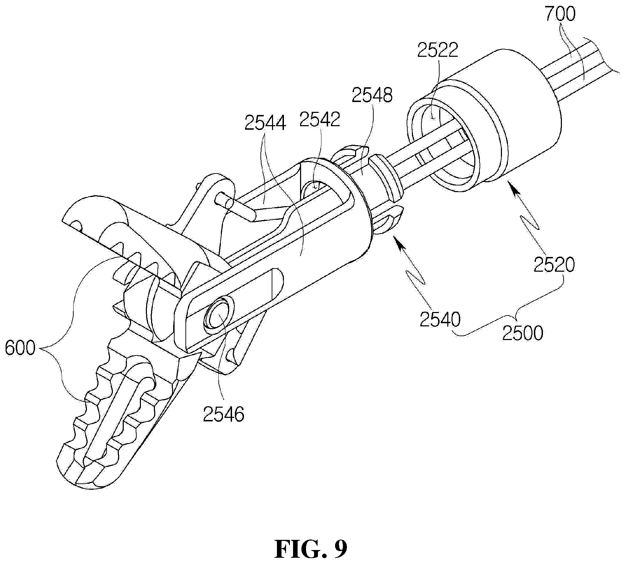

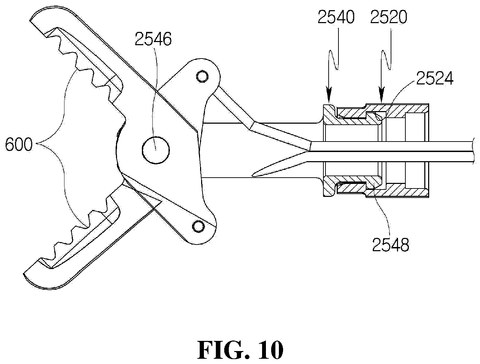

Next, hemostatic forceps for an endoscope according to another embodiment of the present disclosure will be described with reference to FIGS. 9 and 10. Because the embodiments are identical to each other except for the structures of the housings, a description will be focused on the difference between the embodiments.

A housing 2500 is coupled to the front end of the outer tube 400 and serves to support the pair of pincer pieces 600 so that the pair of pincer pieces 600 is rotatable and openable or closable relative to each other. Specifically, the housing 2500 includes: a first member 2520 coupled to the front end of the outer tube 400; and a second member 2540 coupled to be rotatable relative to the first member 2520, and the pair of pincer pieces 600 is openably and closably installed on the second member 2540.

The first member 2520 has a cylindrical shape having a first hollow portion 2522 and is fixed to the front end of the outer tube 400. The second member 2540 also has a cylindrical shape having a second hollow portion 2542 and includes a pair of arms 2544 extending outward from a cylindrical body while facing each other. The pair of pincer pieces 600 is openably and closably installed on the pair of arms 2544. Specifically, the pair of pincer pieces 600 is installed to be rotatable side by side by a pin 2546 installed to simultaneously penetrate the pair of arms 2544. The second member 2540 further includes a plurality of hook parts 2548 protruding toward the first member 2520. The plurality of hook parts 2548 is coupled to a stepped portion 2524 formed on an inner surface of the first member 2520. The stepped portion 2524 may be formed on the inner surface of the first member 2520 so that a height of the stepped portion 2524 increases forward. Because the plurality of hook parts 2548 of the second member 2540 is coupled to the stepped portion 2524, the second member 2540 may be rotatably supported without separating forward from the first member 2520.

Likewise, a surface of the first member 2520 and a surface of the second member 2540, which face each other in an axial direction, may be spaced apart from each other. Therefore, friction may be minimized when the second member 2540 rotates relative to the first member 2520.

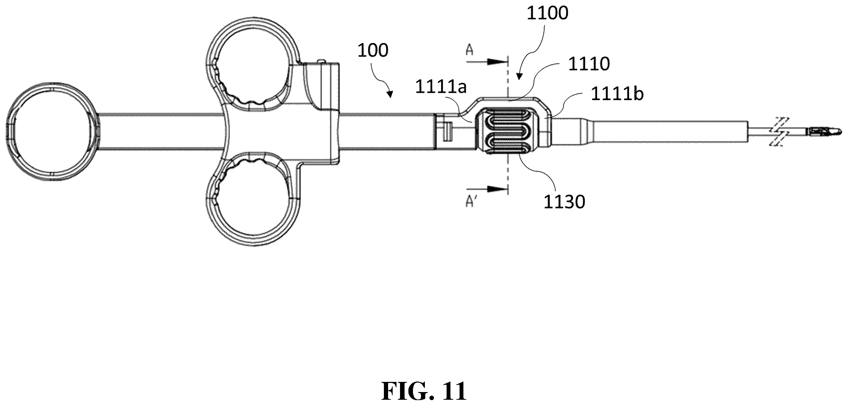

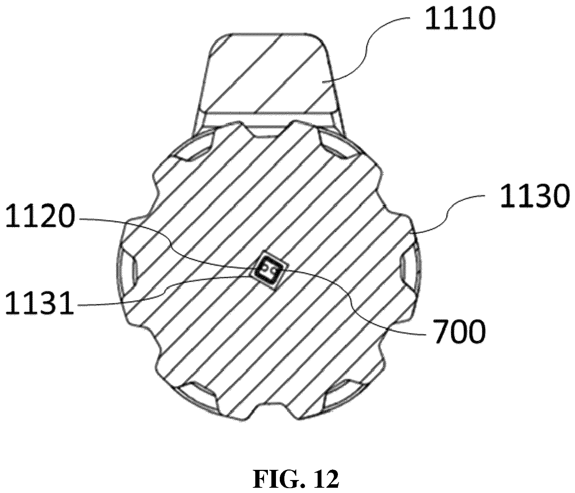

Next, hemostatic forceps for an endoscope according to still another embodiment of the present disclosure will be described with reference to FIGS. 11 and 12. Because still another embodiment is identical to the above-mentioned embodiment except for a configuration in which the fluid injection part 300 is replaced with a rotary dial part 1100, the following description will be focused on the rotary dial part 1100.

The rotary dial part 1100 is coupled to the front end of the handle part 100 and serves to rotate the pair of pincer pieces 600 in conjunction with the pair of wires 700. The rotary dial part 1100 includes a holder part 1110 fixedly coupled to the handle part 100 and having a pair of holder arms 1111a and 1111b spaced apart from each other. A pipe 1120 is connected to and penetrates the pair of holder arms 1111a and 1111b of the holder part 1110. A rotary dial 1130 is rotatably disposed between the pair of holder arms 1111a and 1111b of the holder part 1110. The pipe 1120 is disposed in the rotary dial 1130.

More specifically, as illustrated in FIG. 12, the rotary dial 1130 has a through-hole 1131 provided in a longitudinal direction. The pipe 1120 is disposed in the through-hole 1131, and the pipe 1120 rotates together with the rotary dial 1130 when the rotary dial 1130 rotates. In this case, as illustrated in the drawings, the through-hole 1131 may have a quadrangular shape. In this case, a cross-section of the pipe 1120 may also have a quadrangular shape, such that the pipe 1120 may be disposed and fitted into the through-hole 1131. Of course, the through-hole 1131 and the pipe 1120 may each have another polygonal shape. However, cross-sectional shapes of the two members need to be substantially identical to each other, such that the pipe 1120 rotates together with the rotary dial 1130 when the rotary dial 1130 rotates.

In this case, the pair of wires 700 is coupled to the pipe 1120 and rotated together with the pipe 1120 by the rotation of the pipe 1120. In this case, as illustrated in FIG. 12, the pair of wires 700 may be coupled by being compressed in the pipe 1120. With this configuration, when the rotary dial 1130 rotates, the pipe 1120 in the rotary dial 1130 rotates, the pair of wires 700 connected to the pipe rotates, and as a result, the pair of pincer pieces 600 rotates.

Meanwhile, a concave-convex portion may be provided on an outer surface of the rotary dial 1130, thereby preventing the rotary dial 1130 from slipping when the user holds and rotates the rotary dial.

The present disclosure is not limited to the specific exemplary embodiments and descriptions, various modifications can be made by any person skilled in the art to which the present disclosure pertains without departing from the subject matter of the present disclosure as claimed in the claims, and the modifications are within the protection scope of the present disclosure.

### [Description of Reference Numerals]

1: Hemostatic forceps for endoscope
100: Handle part
102: Handle ring
200: Slider
202: Slider ring
220: Plug
300: Fluid injection part
320: Fluid injection port
400: Outer tube
420: Coil
500, 2500: Housing
520, 2520: First member
522, 2522: First hollow portion
524, 2524: Stepped portion
540, 2540: Second member
542, 2542: Second hollow portion
544, 2544: Pair of arms
546, 2546: Pin
560: Connection member
562: Third hollow portion
580: Separating plate
600, 600a, 600b: Pincer piece
602a, 602b: Closing surface
700, 700a, 700b: Wire
800: Inner tube
900: Connector
920: Sealing part
1000: Protection tube
2548: Hook part
1100: Rotary dial part
1110: Holder part
1111a, 1111b: Pair of holder arms
1120: Pipe
1130: Rotary dial

## Claims

1. Hemostatic forceps for an endoscope, comprising:
a handle part;
a slider slidably installed on the handle part;
a fluid injection part connected to the handle part and having a fluid injection port;
an outer tube extending from the fluid injection part;
a housing coupled to an end of the outer tube;
a pair of pincer pieces installed on the housing so as to be openable and closable relative to each other;
a pair of wires each having two opposite ends respectively connected to the pair of pincer pieces and the slider; and
an inner tube disposed in the outer tube and configured to surround the pair of wires.

2. The hemostatic forceps of claim 1, wherein the housing comprises:
a first member coupled to an end of the outer tube; and
a second member rotatably coupled to the first member, and
wherein the pair of pincer pieces is installed on the second member so as to be openable and closable relative to each other.

3. The hemostatic forceps of claim 2, wherein the second member is rotatably coupled to the first member through a connection member, one end of the connection member is fixedly coupled to an inner side of the second member, and another end of the connection member is coupled to an inner stepped portion of the first member.

4. The hemostatic forceps of claim 2, wherein the second member comprises a plurality of hook parts protruding toward the first member, and the plurality of hook parts is coupled to an inner stepped portion of the first member.

5. The hemostatic forceps of claim 3, wherein the inner stepped portion of the first member is formed on an inner surface of the first member such that a height of the inner stepped portion increases in a direction toward the second member.

6. The hemostatic forceps of claim 2, wherein a surface of the first member and a surface of the second member, which face each other in an axial direction, are spaced apart from each other.

7. The hemostatic forceps of claim 1, wherein the inner tube is disposed to be spaced apart from an end of the housing at a predetermined distance.

8. The hemostatic forceps of claim 1, wherein a fluid injected through the fluid injection port flows through a space between the outer tube and the inner tube and is sprayed through a hollow portion of the housing.

9. The hemostatic forceps of claim 1, wherein a coil is wound around an inner surface of the outer tube.

10. The hemostatic forceps of claim 8, wherein a protection tube is coupled to an end of the fluid injection part and configured to surround the outer tube.

11. Hemostatic forceps for an endoscope, comprising:
a handle part;
a slider slidably installed on the handle part;
a rotary dial part connected to the handle part;
an outer tube extending from the rotary dial part;
a housing coupled to an end of the outer tube;
a pair of pincer pieces installed on the housing so as to be openable and closable relative to each other;
a pair of wires each having two opposite ends respectively connected to the pair of pincer pieces and the slider; and
an inner tube disposed in the outer tube and configured to surround the pair of wires.

12. The hemostatic forceps of claim 11, wherein the rotary dial part comprises:
a holder part fixedly coupled to the handle part and having a pair of holder arms spaced apart from each other;
a pipe connected to the pair of holder arms of the holder part while penetrating the pair of holder arms; and
a rotary dial rotatably disposed between the pair of holder arms of the holder, and
wherein the pipe is disposed in the rotary dial.

13. The hemostatic forceps of claim 12, wherein the rotary dial has a through-hole in a longitudinal direction, and the pipe is disposed in the through-hole and rotates together with the rotary dial when the rotary dial rotates.

14. The hemostatic forceps of claim 13, wherein the through-hole of the rotary dial has a polygonal shape, and a cross-section of the pipe has a polygonal shape substantially identical to the polygonal shape of the through-hole.

15. The hemostatic forceps of claim 13, wherein the pair of wires is connected to the pipe and rotated together with the pipe by a rotation of the pipe and configured to rotate the pair of pincer pieces coupled to distal ends of the pair of wires.
